# EUROPEAN PATENT APPLICATION

(11) **EP 4 197 553 A2**
(43) Date of publication of application: **21.06.2023**
(21) Application number: 21856312.0
(22) Date of filing: 11.08.2021
(51) Int. Cl.: A61K 39/285, A61K 47/18, A61K 47/26

(54) **USE OF COLLAGEN-POLYVINYLPYRROLIDONE IN PULMONARY INFLAMMATION AND FIBROSIS IN PATIENTS INFECTED BY THE SARS-COV2 VIRUS (COVID-19)**

(30) Priority: 12.08.2020 MX 2020008473
(71) Applicant: Aspid, S.A. de C.V., 03300 México (MX)
(72) Inventor: FURUZAWA CARBALLEDA, Guadalupe Janette, Colonia Portales 03300, Ciudad de México (MX)
(74) Representative: Jacobacci Coralis Harle
(86) International application number: PCT/MX2021/050045
(87) International publication number: WO 2022/035306

(57) **Abstract**

The present invention relates to the use of type I polymerized collagen in Covid-19 positive patients and to an intramuscular dosing regimen every 12 h for 3 days and then every 24 h for 4 days in patients with mild to moderate Covid-19 disease, or an intramuscular dosing regimen every 12 h for 7 days in patients with severe to semi-critical illness due to Covid-19. In addition, the clinical evolution of each patient was analyzed individually (Figures 13 to 17 and 20 to 21) and in groups (Figures 18 and 19), taking into account the following parameters: percentage of SpO2, temperature, heart rate, respiratory rate, cough intervals, chest pain, headache, dyspnea, odynophagia, anosmia, loss of sense of taste, presence of arthralgia and myalgia, and clinical improvement was documented in all cases.

## Description

### TECHNICAL FIELD

### Background

At the present time the world is in a health emergency due to the presence of a massive infection by the SARS-CoV2 virus.

Despite all the efforts of the medical and scientific community in the health field, no curative treatment or that destroys the virus has been found.

Based on the foregoing, innumerable efforts have been made to help patients affected by this condition to correct the pathological state and its possible sequelae.

The infection caused by the SARS-CoV2 virus produces massive cell destruction in the lung and other organs, which is accompanied by a state of hyperinflammation, which causes pulmonary fibrosis in recovered patients and death in those patients with multiple organ failure.

In an effort to control the inflammatory process that leads to alveolar destruction and fibrosis, with the consequent loss of function of the respiratory organ, the effect of the anti-IL-6 receptor monoclonal antibodies (tocilizumab) and the use of mesenchymal precursor cells is being evaluated. The common denominator of these studies is to modulate the inflammatory response, thereby shortening the period of status and potentially limiting the development of pulmonary fibrosis.

The role of some immunosuppressants such as corticosteroids, colchicine, and inhibitors of the JAK/STAT signaling pathway is also being evaluated as a resource to modulate the immune response.

Based on the above information, the use of an immunoregulator, rather than an immunosuppressant, such as COLLAGEN-POLYVINYLPYRROLIDONE, (Fibroquel registered trademark; SSA code: 010 000 3999), which has a mechanism of action that in a relevant way: negatively modulates the expression of IL-1β, IL-8, TNF-α, TGF-β1, IL-17, Cox-1, leukocyte adhesion molecules (ELAM-1, VCAM-1 and ICAM-1), significantly increases the mediators and inflammation modulating mechanisms (IL-10 expression and the number of regulatory T cells); likewise, it reduces tissue fibrosis, and would be relevant in controlling the cytokine storm that occurs due to hyperinflammation caused by macrophage activation syndrome (secondary hemophagocytic lymphohistiocytosis) .

It is important to highlight that collagen-polyvinylpyrrolidone (collagen-PVP) was protected in Mexican patent No. 264 089 and its counterparts both in Europe and in other jurisdictions. The '089 patent describes and claims the use of collagen-PVP to modulate chronic joint inflammation, wherein all the analyzes of rheumatoid arthritis (RA) and osteoarthritis (OA) are discussed in detail from different approaches that cover clinical, pathological, histological, biomechanical, biochemical and immunological aspects of the tissues of the affected joint cavity and very particularly, both RA and OA were studied considering them autoimmune and non-autoimmune inflammatory processes, respectively; a drug based on collagen-PVP was proposed to be used in the treatment of these diseases and its cellular interactions in the human body were analyzed to avoid undesirable side effects such as toxicity and immunogenicity and looking for alternatives to avoid surgery.

The regulatory mechanism of inflammation exerted by type I polymerized collagen is supported by 2 models: the first, non-autoimmune inflammation [osteoarthritis (OA) model] and the second, autoimmune inflammation [rheumatoid arthritis (RA) model)]

### Non-autoimmune (OA) and autoimmune (AR) inflammation model:

Cartilage and synovium co-cultures were performed from 9 patients with RA and 8 patients with OA who underwent hip or knee replacement (Table 1). Joint tissue was cultured with **(a)** RPMI (control), **(b)** 1% type I polymerized collagen, for 7 days. The supernatants and tissues of days 1 and 7 were collected. In the culture supernatants the concentration of IL-1β, IL-8, IL-10, IL-12, TNF-α and IFN-γ was quantified by ELISA and the results were normalized with total protein concentration. The expression of IL-1β and TNF-α was evaluated using histochemistry.

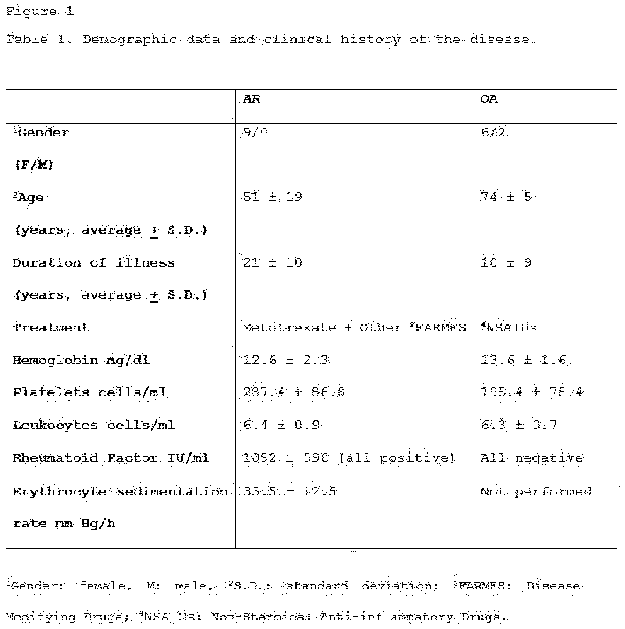

The demographic and clinical data of the patients are detailed in Figure 1.

*Synthesis of pro-inflammatory cytokines in cartilage and synovial co-cultures from patients with RA or OA.*

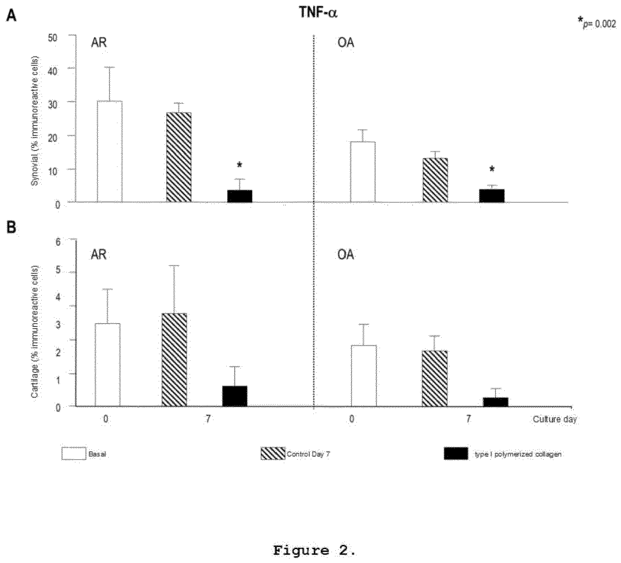

A large number of cytokines (pro- and anti-inflammatory) and growth factors are involved in the pathophysiology of OA and RA. Among the pro-inflammatory cytokines that have been shown to play a pivotal role in the development of these diseases are IL-1β and TNF-α. The addition of type I polymerized collagen to the RA or OA co-cultures decreased the tissue expression of TNF-α vs. controls in synovial tissue (Fig. 2).

**Figure 2****. Effect of type I polymerized collagen on TNF-α expression. (A)** Cells immunoreactive to TNF-α in the synovial tissue of the co-cultures. **(B)** Immunoreactive TNF-α+ cells in cartilage from co-cultures. Rheumatoid arthritis (RA): left panel. Osteoarthritis (OA): right panel. Basal: tissue in basal conditions. Control Day 7: control tissue grown with RPMI for 7 days and that did not receive any treatment. Each culture was performed in triplicate for each condition, of which at least two sections of each of the tissues were analyzed. The results are expressed as the mean ± standard error (SEM) of the percentage of immunoreactive cells in 9 co-cultures of patients with RA and 8 with OA. **p*≤0.002.

*Levels of pro-inflammatory cytokines in cartilage and synovial co-culture supernatants from patients with RA or OA*.

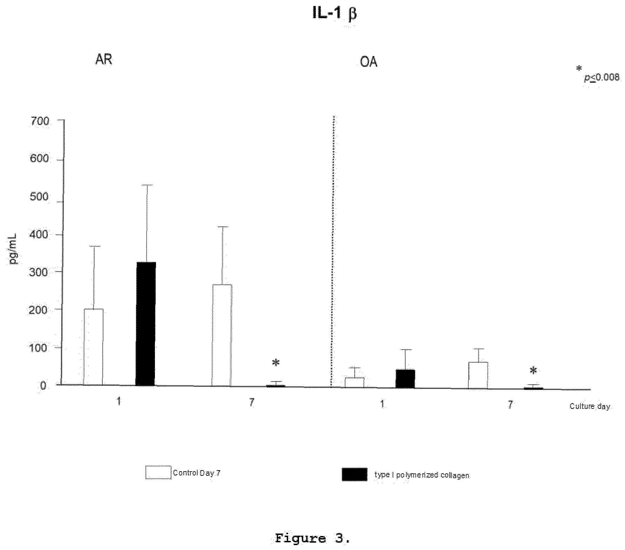

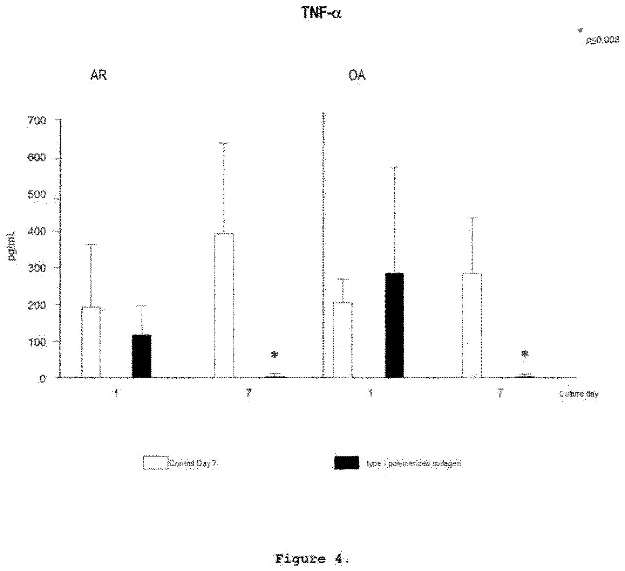

Likewise, the addition of type I polymerized collagen to the AR or OA co-cultures decreased the expression of IL-1β and TNF-α vs. the controls in the supernatants at day 7 of culture (Fig. 3 and Fig. 4, respectively).

**Figure 3****. Effect of type I polymerized collagen on IL-1β expression in supernatants of cartilage and synovium co-cultures from patients with rheumatoid arthritis (RA) or osteoarthritis (OA).** The data represent the mean ± SD of 9 co-cultures of cartilage and synovial tissue from patients with RA and of 8 co-cultures of patients with OA. Each culture was performed in triplicate for each condition. The results were normalized with the total protein concentration of the supernatant, determined by the Folin-Löwry micromethod. AR: left panel. OA: panel on the right. Basal: tissue in basal conditions. Control Day 7: control tissue grown with RPMI for 7 days and that did not receive any treatment. **p*≤0.008.

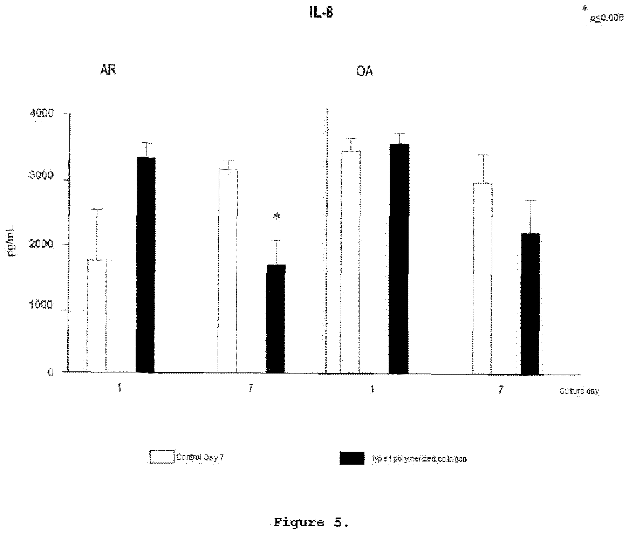

**Figure 4****. Effect of type I polymerized collagen on TNF-α expression in supernatants of cartilage and synovial tissue co-cultures from patients with rheumatoid arthritis (RA) or osteoarthritis (OA).** The data represent the mean ± SD of 9 co-cultures of cartilage and synovium from patients with RA and of 8 co-cultures of patients with OA. Each culture was performed in triplicate for each condition. The results were normalized with the total protein concentration of the supernatant, determined by the Folin-Löwry micromethod. AR: left panel. OA: panel on the right. Basal: tissue in basal conditions. Control Day 7: control tissue grown with RPMI for 7 days and that did not receive any treatment **p*≤0.008. A similar pattern was observed in relation to the concentration of IL-8 in the supernatants of the co-cultures (Fig. 5). However, the greatest decrease was determined in tissue co-cultures from RA patients treated with type I polymerized collagen. No detectable levels of IL-12 and IFN-γ were determined in the cultures.

F**igure 5. Effect of type I polymerized collagen on IL-8 expression in supernatants of cartilage and synovial tissue co-cultures from patients with rheumatoid arthritis (RA) or osteoarthritis (OA).** The data represent the mean ± SD of 9 co-cultures of cartilage and synovium from patients with RA and of 8 co-cultures of patients with OA. Each culture was performed in triplicate for each condition. The results were normalized with the total protein concentration of the supernatant, determined by the Folin-Löwry micromethod. AR: left panel. OA: panel on the right. Basal: tissue in basal conditions. Control Day 7: control tissue grown with RPMI for 7 days and that did not receive any treatment. **p*≤0.006.

*Levels of anti-inflammatory factors in cartilage and synovial co-culture supernatants from patients with* RA *or OA.*

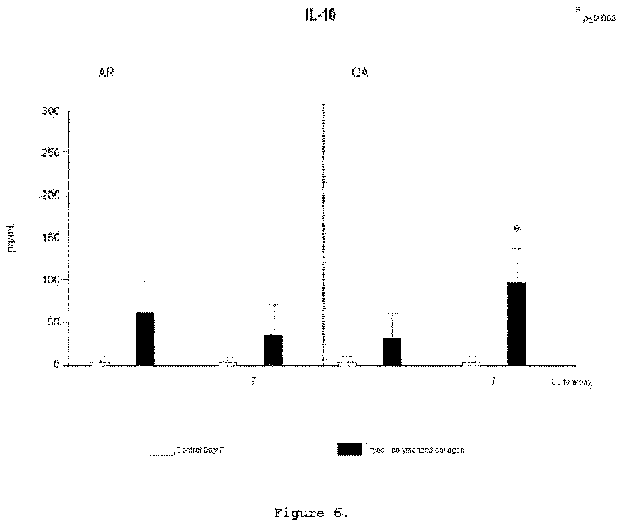

The addition of type I polymerized collagen to the RA or OA co-cultures increased the IL-10 concentration between 10 and 25 times in the supernatants of the co-cultures, to statistically significant levels at day 7 vs. on day 1 of culture (Fig. 6).

**Figure 6****. Effect of type I polymerized collagen on IL-10 expression in supernatants of cartilage and synovial tissue co-cultures from patients with rheumatoid arthritis (RA) or osteoarthritis (OA).** The data represent the mean ± SD of 9 co-cultures of cartilage and synovium from patients with RA and of 8 co-cultures of patients with OA. Each culture was performed in triplicate for each condition. The results were normalized with the total protein concentration of the supernatant, determined by the Folin-Löwry micromethod. AR: left panel. OA: panel on the right. Basal: tissue in basal conditions. Control Day 7: control tissue grown with RPMI for 7 days and that did not receive any treatment. **p*≤0.006. *Gene expression of pro- and anti-inflammatory cytokines in the tissues of cartilage and synovial co-cultures from patients with RA or OA.*

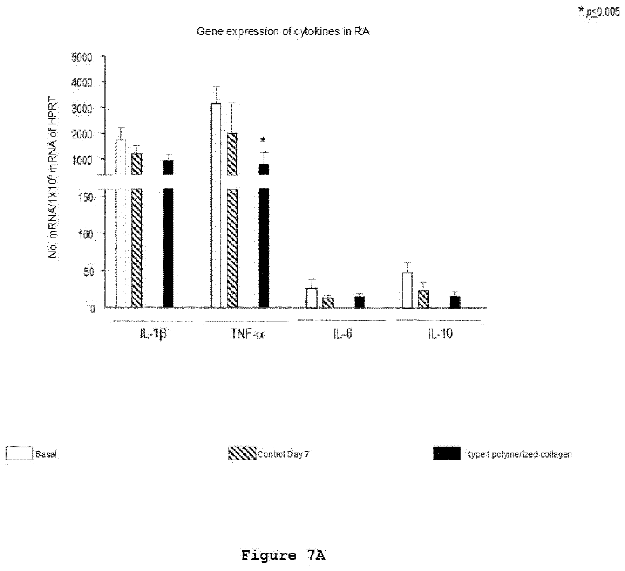

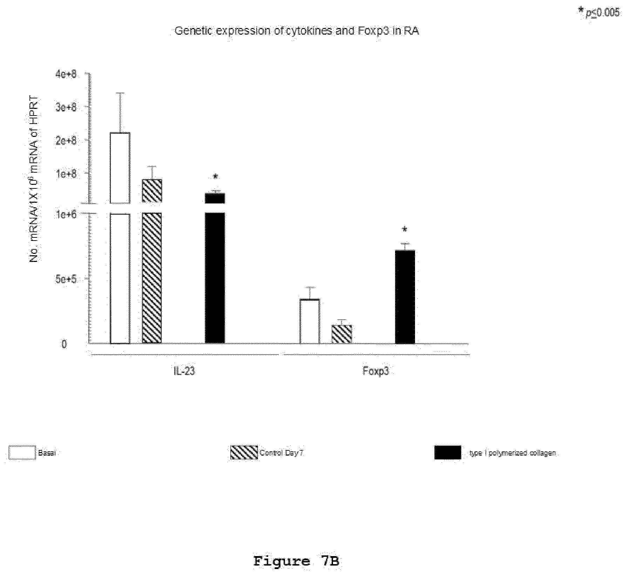

The evaluation of the gene expression of pro- and anti-inflammatory cytokines showed that type I polymerized collagen added to joint tissue co-cultures from patients with RA negatively regulated the transcription of genes such as TNF-α and IL-23 (Fig. 7A, B) and positively that of regulatory T cells, FOXP3, at statistically significant levels with respect to the control (Fig. 7B).

**Figure 7****. Effect of type I polymerized collagen on the gene expression of pro- and anti-inflammatory cytokines and factors in the tissues of cartilage and synovial tissue co-cultures of patients with rheumatoid arthritis (RA). (A)** Quantification of IL-1β, TNF-α, IL-6 and IL-10 gene expression and **(B)** Quantification of IL-23 and Foxp3 gene expression by RT-PCR in synovial membrane tissues and cartilage co-cultured for 7 days. The bars show the mean ± SD of the transcript levels of the samples normalized through the expression of the housekeeping gene GADPH determined by 2ΔΔCt. The data represent the mean ± SD of 9 cartilage and synovial co-cultures from RA patients. Each culture was performed in triplicate for each condition. Basal: tissue in basal conditions. Control Day 7: control tissue grown with RPMI for 7 days and that did not receive any treatment. *p values ≤0.05 were considered significant.

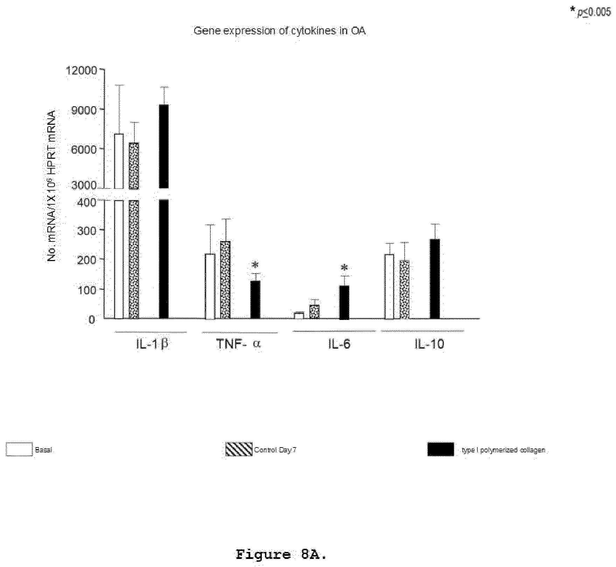

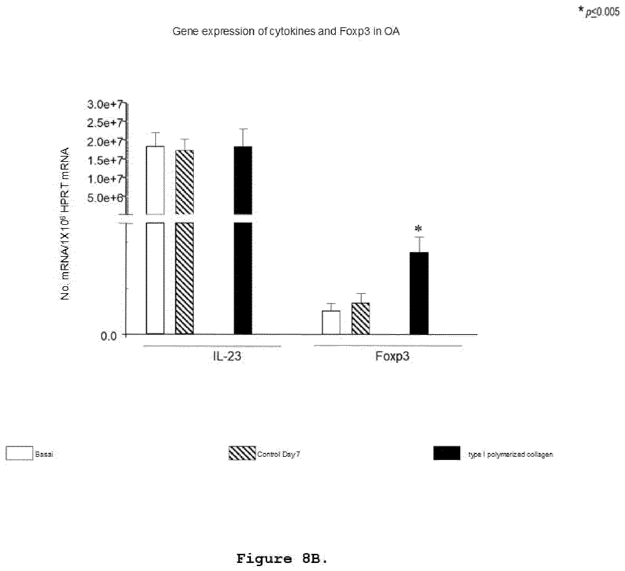

While the addition of type I polymerized collagen to joint tissue co-cultures from patients with OA induced a negative regulation of the gene expression of TNF-α and a positive regulation of Foxp3 at statistically significant levels with respect to the control (Fig. 8) .

**Figure 8****. Effect of type I polymerized collagen on the gene expression of pro- and anti-inflammatory cytokines and factors in the tissues of cartilage and synovial co-cultures of patients with Osteoarthritis (OA). (A)** Quantification of IL-1β, TNF-α, IL-6 and IL-10 gene expression, **(B)** IL-23 and Foxp3 by RT-PCR in co-cultured cartilage and synovial membrane tissues for 7 days. The bars show the mean ± SD of the transcript levels of the samples normalized through the expression of the housekeeping gene GADPH determined by 2ΔΔCt. The data represent the mean ± SD of 8 co-cultures from patients with OA. Each culture was performed in triplicate for each condition. Basal: tissue in basal conditions. Control Day 7: control tissue grown with RPMI for 7 days and that did not receive any treatment. **p* values ≤ 0.05 were considered significant.

In the present study, we demonstrated that the addition of type I polymerized collagen to cartilage and synovial co-cultures from patients with RA decreased the synthesis of pro-inflammatory cytokines such as IL-1β, IL-8, IL-23 and TNF-α at the gene and protein level, which could be directly or indirectly related to the increase observed in the regulatory mechanisms of inflammation, mediated by the increase in IL-10 levels and Foxp3 gene expression in regulatory T cells.

In both diseases, the synovial membrane, but not the cartilage, seems to be the tissue that contributes most significantly to the synthesis of soluble pro-inflammatory factors.

The action of type I polymerized collagen seems to depend on the cell activation stage as demonstrated by Jou I-M, et al, 2005 and Furuzawa-Carballeda et al, 2003 since the addition of type I polymeric atelopeptide collagen to dormant cells does not induce any response, that is, it has no effect. The results obtained are consistent with the analysis of various extracellular matrix proteins that have been used as potentially therapeutic molecules in animal models of:
a) autoimmune diseases, such as RA in which synthetic fibronectin peptides have been evaluated to inhibit or interrupt the infiltration of inflammatory cells into the joint cavity (Hines KL, 1994; Wahl SM, 1994), or thrombospondin-1 (TSP-1) and angiostatin (plasminogen fragments) as a strategy to inhibit angiogenesis (Yin G, 2002; Jou I-M, 2005).
b) Suppression or inhibition of tumor growth and metastasis, such is the case of endostatin (collagen XVIII fragments)-angiostatin and soluble Tie-2 that increase the suppression of prostate tumor growth and melanoma (Scappaticci FA, 2001; Raikwar SP, 2005) or complexes of endostatin with DNA within cationic liposomes to prevent osteosarcoma metastasis (Peterszegi G, 1998; Pasco S, 2003; Honerbeck W, 2003; Maquart FX, 2004; Bellon F, 2004; Duca L, 2004; Pasco S, 2004; Maquart FX, 2005).

In conclusion, type I polymerized collagen induces the negative regulation of the expression of some pro-inflammatory cytokines, stimulates the production of anti-inflammatory cytokines and the expression of Foxp3 in regulatory T cells, favoring the mechanisms of negative modulation of inflammation.

### Autoimmune inflammation (AR) model. Evaluation of the regulatory effect of inflammation, inducer of immunological tolerance and of the mechanism of action of type I polymerized collagen in a murine model of collagen-induced arthritis (CIA)

The objective of the study was to evaluate whether the intramuscular application of type I polymerized collagen or type I polymerized collagen combined with methotrexate (MTX) was capable of preventing the establishment or progression of collagen injection-induced arthritis (CIA) in rodents. The effect was determined at the clinical, histological and molecular level by comparing the results with the gold standard treatment: MTX.

For this, 3 models were carried out:
*Toxicity Model.* Twenty-one 8-week-old male mice of the DBA1/OlaHsd strain were treated with 100 µl of **(a)** placebo (citrate buffer), **(b)** type I polymerized collagen, **(c)** MTX (2.5mg/kg), **(d)** Type I polymerized collagen (b) with MTX (c) (vol:vol), weekly for six weeks. Three animals were euthanized in the sixth week and three in the thirteenth week. The spleen, lymph nodes, joint tissue, liver, lungs, kidneys and heart were obtained in order to assess the inflammatory infiltrate, the predominant cell populations and the expression of pro- and anti-inflammatory cytokines. *Early Arthritis Model.* Fifty mice with the same characteristics were immunized at the base of the tail with 100 µg of chicken type II collagen emulsified in complete Freund's adjuvant. The boost was applied on day 21 at the base of the tail with 100 µg of type II collagen emulsified with Freund's incomplete adjuvant. On the same boosting day, the treatments **(a-d)** were applied every week for six weeks. Three animals were euthanized in the sixth week and three in the thirteenth week. The spleen, lymph nodes, joint tissue, liver, lungs, kidneys and heart were obtained in order to assess the inflammatory infiltrate, the predominant cell populations and the expression of pro- and anti-inflammatory cytokines.

*Established Arthritis Model.* Arthritis was induced as in the previous model in 50 mice and the treatments were applied intramuscularly, after 2 weeks of the boost, every week for 6 weeks. Three animals were euthanized in the eighth week and three in the fifteenth week. The spleen, lymph nodes, joint tissue, liver, lungs, kidneys and heart were obtained in order to assess the inflammatory infiltrate, the predominant cell populations and the expression of pro- and anti-inflammatory cytokines.

*Histological evaluation.* The inflammatory infiltrate and tissue architecture were evaluated by H&E and Massón's trichrome staining and the content of proteoglycans, with the PAS technique.

*Cytometric evaluation.* Using FACS, the percentage of populations of Th1 (CD4⁺/IFN-γ⁺), Th2 (CD4⁺/IL-4⁺), Treg (CD4⁺/FOXP3⁺) Th17 (CD4⁺/IL-17⁺) and dendritic (CD4-/CD11c⁺) cells in spleen cells.

*Effect of type I polymerized collagen and the mixture of type I polymerized collagen and methotrexate (MTX) in the toxicity model.*

None of the treatments evaluated in the study produced toxicity in the mice. It was evaluated by gross and histopathological analysis of the kidneys, heart, lungs, spleen, lymph nodes, and hind legs. All tissues analyzed had a normal architecture. No inflammatory infiltrates or other abnormalities were observed.

*Histopathological analysis of the effect of type I polymerized collagen in the early (preventive) and long-standing (late or palliative) arthritis model.*

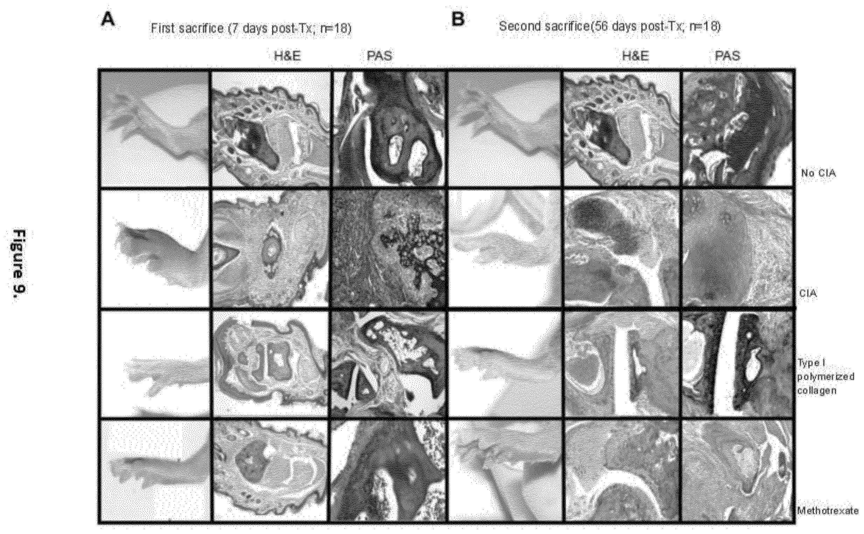

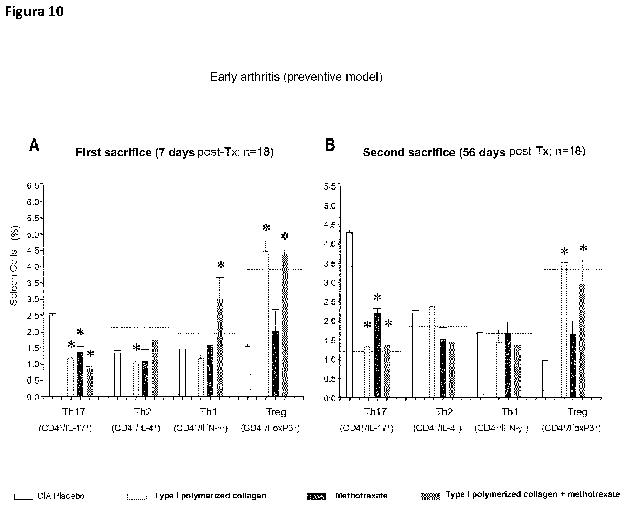

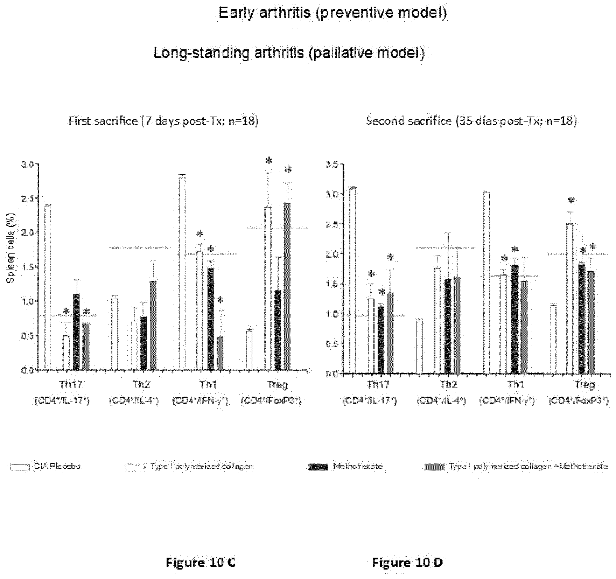

Histopathological analysis was performed on the hind legs of mice with CIA, by two investigators in a blinded fashion. Representative images of H&E and PAS stained joint tissue sections from the CIA groups, treated with **(a)** placebo, **(b)** type I polymerized collagen, **(c)** MTX are presented in Figure 9. CIA mice showed typical arthritis, which is characterized by extensive infiltration of inflammatory cells, synovial hyperplasia, loss of joint space, and bone erosion. Treatment with type I polymerized collagen decreased the degree of inflammation and preserved the joint structure. While treatment with polymerized atelopeptide type I collagen/MTX resulted in a significant reduction of inflammatory infiltrates and recovery of tissue architecture. MTX induced some tissue abnormalities, such as the presence of nodules (amorphous fibrin tissue) and poor quality lesion repair tissue, as well as inflammatory infiltrates. The effect of the different conditions was maintained until the second sacrifice (Fig. 10).

**Figure 9****. Palliative effect of type I polymerized collagen on histological damage in mice with CIA.** Hematoxylin and Eosin and PAS staining. **(A)** Representative section of the first sacrifice. **(B)** Representative section of the second sacrifice. The magnification or amplification of the histologies corresponds to 100X.

*Effect of type I polymerized collagen in the early (preventive) and long-standing (late or palliative) arthritis model on the percentage of spleen CD4+ T cell subpopulations.*

The percentage of spleen Th17 cells in mice without CIA (controls) was ~1.3%, while in mice with CIA in the early arthritis model it was 2.5% and 4.3%, for the first and second sacrifice, respectively (Fig. 10A and B) and 2.4% and 3.6%, for the first and second sacrifice, respectively in the long-standing arthritis model (Fig. 10C and D).

Type I polymerized collagen **(b),** MTX **(c)** and the combination of both **(d),** produced a sustained and statistically significant reduction, which maintained the levels of pro-inflammatory cells, Th17, in ranges considered normal, both in the early arthritis model during the first and second sacrifice (1.0-1.4% and 0.9-1.35%, respectively, Fig. 10A and B), and in the long-standing arthritis model during the first and second sacrifice (0.5- 0.45% and 0.9-1.45%, respectively, Fig. 10C and D).

The number of circulating Th2 cells was modified only during the first sacrifice with the polymerized collagen type I treatment **(b)** in the early arthritis model (Fig. 10D).

It should be noted that the treatment of early and long-standing arthritis with type I polymerized collagen **(b)** and the mixture combined with MTX **(d)** induced an increase in Foxp3+ regulatory T cells to statistically significant levels compared to those of mice with arthritis treated with **(a)** placebo or vehicle, and higher than those of healthy mice in both the first and second sacrifice (Fig. 10A, B, C and D). While **(c)** MTX induced a significant reduction in the percentage of these cells in both models.

Regarding regulatory cells, formerly known as suppressor T cells (5% to 15% of peripheral and tissue CD4+ T cells in healthy individuals), they exert extrinsic cellular immunosuppression and tolerance to self and foreign antigens. Tregs modulate the natural course of immune responses, protecting tissues from inflammation by limiting damage and preventing autoimmunity. They suppress immune responses by producing granzymes and perforins, depleting IL-2, secreting suppressor molecules such as IL-19 and TGF-β1 and decreasing the functions of antigen presenting cells (APCs), in addition they promote anergy or apoptosis of T cells effectors. As such, Tregs play an important role in tissue repair and homeostasis.

**Figure 10****. Effect of type I polymerized collagen in the early (preventive) and long-standing (late or palliative) arthritis model on the percentage of spleen CD4+ T cell subpopulations. (A)** Spleen cells obtained *ex vivo* in the early arthritis model during the first sacrifice. **(B)** Spleen cells obtained *ex vivo* in the early arthritis model during the second sacrifice. **(C)** Spleen cells obtained *ex vivo* in the long-standing arthritis model during the first sacrifice. **(D)** Spleen cells obtained *ex vivo* in the long-standing arthritis model during the second sacrifice. The intracellular production of IL-17A, IL-4, IFN-γ and Foxp3 by CD4+ T cells was detected by flow cytometry. The results are representative of 6 mice analyzed in each group. The horizontal dotted line represents the average normal values obtained from mice without CIA (n = 3). Data represent mean ± ESM. ∗p <0.05.

*Effect of type I polymerized collagen in the early (preventive) and long-standing (late or palliative) arthritis model on the percentage of CD4-*/*CD11c⁺ dendritic cells in the spleen.*

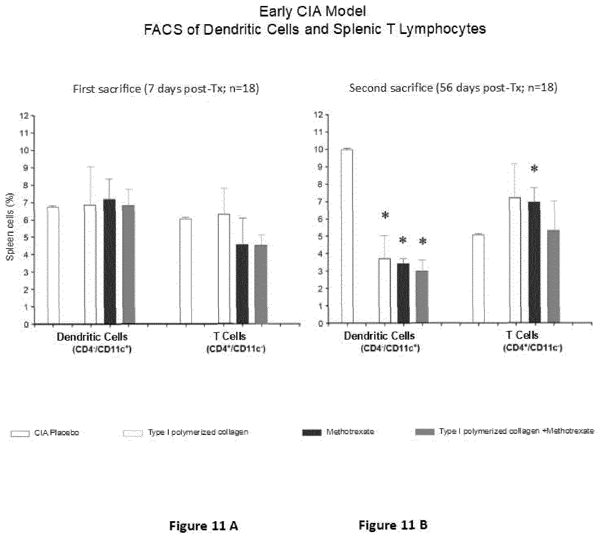

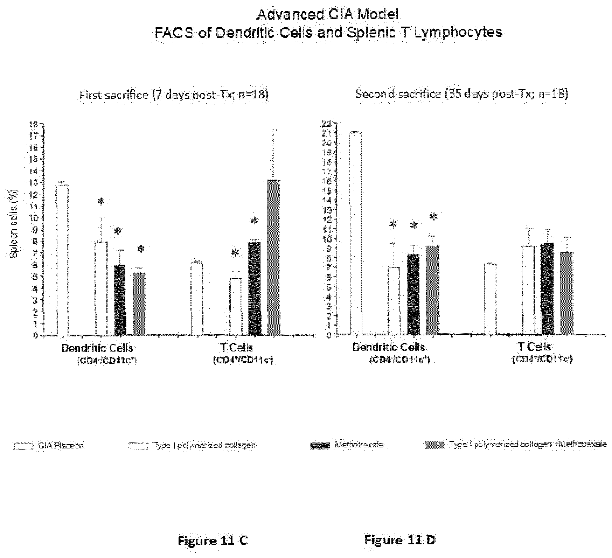

CD11c cells are dendritic cells that link the innate immune response with the adaptive one, through the recruitment of immune effector cells such as NK cells, NKT (natural killer T cells) and neutrophils. In addition, dendritic cells have the main function of presenting antigens and directing the immune response. In the early arthritis model, CD11c cells were presented in a lower and statistically significant percentage in the three treatments with respect to the control during the second sacrifice (Figure 11A). While in the established arthritis model, CD11c cells were present in a lower and statistically significant percentage in the three treatments with respect to the control, both in the first and in the second sacrifice (Figure 11C, D).

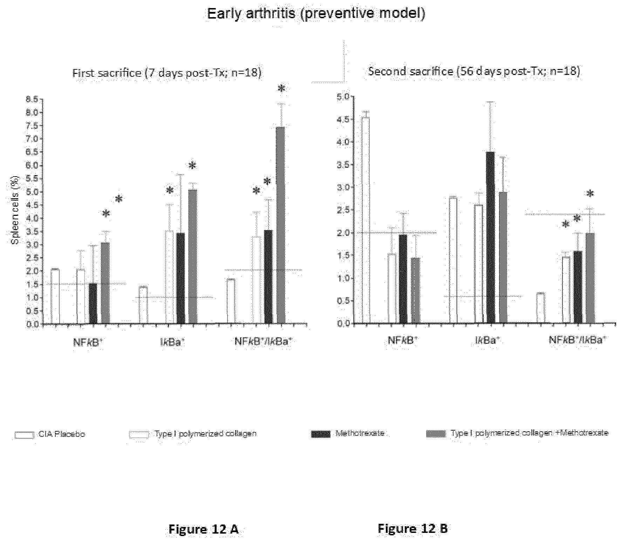

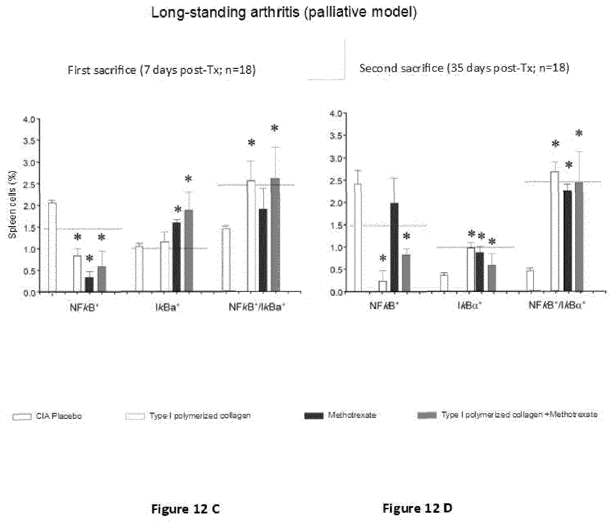

**Figure 12****. Effect of type I polymerized collagen in the early (preventive) and long-standing (late or palliative) arthritis model on the percentage of CD11c⁺ cells in the spleen. (A)** Spleen cells obtained *ex vivo* in the early arthritis model during the first sacrifice. **(B)** Spleen cells obtained *ex vivo* in the early arthritis model during the second sacrifice. **(C)** Spleen cells obtained *ex vivo* in the long-standing arthritis model during the first sacrifice. **(D)** Spleen cells obtained *ex vivo* in the long-standing arthritis model during the second sacrifice. The expression of CD11c was detected by flow cytometry. The results are representative of 6 mice analyzed in each group. The horizontal dotted line represents the average normal values obtained from mice without CIA (n = 3). Data represent mean ± ESM. ∗p <0.05

*Effect of type I polymerized collagen in the early (preventive) and long-standing (late or palliative) arthritis model on the transcription factor NF-kB.*

We infer that the mechanism of action of type I polymerized collagen could be given through the regulation of the transcription factor NF-κB and AP-1. In particular, NF-κB regulates the expression of pro-inflammatory enzymes, cytokines, chemokines, immunoreceptors, and cell adhesion molecules, as well as apoptosis. In the light of this knowledge, the expression of NΓ-κBp65 and its inhibitor IκBα in splenocytes were analyzed *ex vivo* (Fig. 12).

In mice with CIA, the percentage of NF-kB+ and IκBα+ splenocytes was higher, while that of NF-kB+/IκBα+ was lower compared to mice without CIA and to the treatments administered, especially that of polymerized collagen type I or the mixture combined with MTX in both the early and long-standing arthritis models (Fig. 12). The percentage of NF-kB+ complexes with its inhibitor IκBα+ increased to a statistically significant level in those groups of mice with early and long-standing arthritis under the treatments with polymerized collagen type I (treatment **b**) and MTX with polymerized collagen type I (treatment **d**), which suggests a negative regulation of the transcription factor and consequently of inflammation.

**Figure 12****. Effect of type I polymerized collagen in the early (preventive) and long-standing (late or palliative) arthritis model on *ex vivo* NF-κB and IκB-α expression in splenocytes. (A)** Spleen cells from early arthritis model mice at first sacrifice. **(B)** Splenocytes obtained immediately after the second sacrifice. **(C)** Spleen cells from long-standing arthritis model mice at first sacrifice. **(D)** Splenocytes obtained immediately after the second sacrifice. The intracellular levels of NF-κBp65 and IκBα cells were detected by flow cytometry. The results are representative of 6 mice analyzed in each group. The horizontal dotted line represents the average normal values obtained from mice (n = 3) without CIA. Data represent mean ± SEM. ∗*p* <0.05.

### Adverse effects.

There was no evidence of adverse events after injection of placebo or the different treatments throughout the study. The only adverse event observed was pain lasting less than 15 minutes at the injection site.

In conclusion, with the toxicity model it was possible to verify that there was no damage to the analyzed organs, nor any toxicity with any of the evaluated conditions.

The study demonstrated that both **(b)** polymerized type I collagen, as monotherapy, as well as the combination of **(d)** polymerized type I collagen with MTX exhibit both preventive and palliative effects in the CIA model, through the negative regulation of antigenic presentation (decrease in the CD11c⁺ population), of the subpopulation of Th17 cells (1.5-2.0X and 3.0-4.0X, respectively) and positive regulation of Tregs cells (5.0-6.0X and 5.5-6.0, respectively). The mechanism of action appears to be directly related to the regulation of the transcription factor NF-kB.

The joint of the mice with CIA treated with **(b)** type I polymerized collagen maintained a tissue architecture similar to normal, without inflammatory infiltrates, bone erosions or loss of joint space, as well as the content of proteoglycans. Type I polymerized collagen treatments were safe and effective. There were no adverse effects.

In view of this background, it is clear that a drug capable of modulating soluble pro- and anti-inflammatory mediators, as well as the number of pro-inflammatory effector cells and inflammation regulatory cells, may be useful to treat different inflammatory, both acute and chronic, as well as autoimmune and non-autoimmune processes, of short or long evolution. However, it is not possible to simply assume that the drug works efficiently in all cases of inflammation, therefore, it is an object of the invention to evaluate the clinical and immunological effect of intramuscular administration of type I polymerized collagen in the cytokine storm in patients with mild to severe and potentially semi-critical disease due to Covid-19.

It is another object of the invention to assess the use of the drug of the invention through the clinical course of patients diagnosed with Covid-19.

It is still another object of the invention to evaluate the clinical effect of the use of type I polymerized collagen drug, administered intramuscularly in patients diagnosed with mild to semi-critical Covid-19 infection.

It is another object of the invention to propose a therapeutic scheme for the type I polymerized collagen drug, administered intramuscularly in patients diagnosed with mild to moderate Covid-19 infection (Table 1; criteria 1-4).

It is another object of the invention to propose a therapeutic scheme of the type I polymerized collagen drug, administered intramuscularly in patients diagnosed with severe to semi-critical Covid-19 infection (Figure 1, Table 1; criteria 5-6) .

It is another object of the invention to demonstrate that collagen-PVP is a drug that has a regulatory effect on cytokine storm both in early and late stages of infection caused by SARS-CoV2 (COVID-19).

Another object of the invention is to establish experimentally and demonstrate that collagen-PVP is a drug that can be used in the hyper inflammatory process observed in the infection caused by the SARS-CoV2 virus (Covid-19) with frankly positive and very important effects, without undesirable side effects as has been demonstrated for over twenty years.

An additional object of the invention is to evaluate at the immunological level, the effect of intramuscular administration of type I polymerized collagen in patients with moderate to severe disease due to SARS-CoV2 (Covid-19). Demonstrate that type I polymerized collagen administered intramuscularly modifies post-pneumonia fibrosis caused by SARS-CoV2 (Covid-19), preventing the patient from developing fibrotic sequelae, without producing adverse effects (based on the results of previous research).

It is worth mentioning that type I polymerized collagen has been developed in Mexico by Mexican researchers and their study has been endorsed and supported by CONACyT on various occasions (CONACyT SALUD 2002-01-7421; CONACyT SALUD 2003-C01-127/B1; CONACyT SALUD 2004-C01-65; INNOVAPYME C0003 2012-01), therefore, it is supported by various studies that are relevant to the current health crisis in Mexico and the world.

### Summary of the Invention

The use of the drug of the invention, that is, the use of polyvinylpyrrolidone collagen (collagen-PVP) was administered intramuscularly, due to its systemic effect, since it could negatively regulate the expression of pro-inflammatory cytokines, leukocyte adhesion molecules, and increasing both IL-10 and the number of regulatory T cells, which can lead to important benefits for the treatment of the hyper inflammatory phase that patients with SARS-CoV2 (Covid-19) present.

If this drug is administered intramuscularly, it could modify post-pneumonia fibrosis due to SARS-CoV2 (Covid-19), by preventing acute respiratory failure syndrome, thus preventing the patient from developing fibrotic sequelae, without producing adverse effects (based on the results of previous research).

The above was evaluated as adjuvant treatment in a two-arm pilot trial, one with intramuscular administration of 1.5 ml of the active ingredient and the other with 1.5 ml vehicle, for 3 days every 12 hours and for 4 days every 24 hours to make a total of 7 days of treatment with a total dose of 15 ml of Collagen-PVP in patients with mild to moderate pneumonia due to Covid-19 (Table 1; category 1 to 4). Clinical improvement, changes in the levels of systemic inflammation and soluble pro-inflammatory mediators, as well as fibrosis were evaluated at 7 and 14 days post-treatment. Likewise, the effect of intramuscular administration of 1.5 ml of the active principle, for 7 days every 12 hours to make a total of 21 ml of Collagen-PVP in patients with severe to semi-critical pneumonia due to Covid-19 (Table 1; category 5 to 6) was also studied.

### Classification of severity of Covid-19 pneumonia

| **Severity category** | **Description** |
|---|---|
| 1 | Not hospitalized, without limitation of activities |
| 2 | Not hospitalized, but with activity limitation and/or supplemental oxygen requirement |
| 3 | Hospitalized, but no need for supplemental oxygen or ongoing medical care (remains hospitalized for isolation) |
| 4 | Hospitalized, no need for supplemental oxygen, but requires ongoing medical care |
| 5 | Hospitalized, requires supplemental oxygen |
| 6 | Hospitalized, requires non-invasive mechanical ventilation or high-flow tips |
| 7 | Hospitalized, requires IMV or ECMO |
| 8 | Death |

| | |
|---|---|
| Beigel JH, 2020 | |

### DESCRIPTION OF THE FIGURES

Figure 1 is a Table with Demographic and Clinical Data in RA and OA.
Figure 2 is a graph of the effect of type I polymerized collagen on TNF-α expression.
Figure 3 is a graph of the effect of type I polymerized collagen on IL-1β expression in supernatants of cartilage and synovial co-cultures from patients with rheumatoid arthritis (RA) or osteoarthritis (OA).
Figure 4 is a graph of the effect of type I polymerized collagen on TNF-α expression in supernatants of cartilage and synovial tissue co-cultures from patients with rheumatoid arthritis (RA) or osteoarthritis (OA).
Figure 5 is a graph of the effect of type I polymerized collagen on IL-8 expression in supernatants of cartilage and synovial tissue co-cultures from patients with rheumatoid arthritis (RA) or osteoarthritis (OA).
Figure 6 is a graph of the effect of type I polymerized collagen on IL-10 expression in supernatants of cartilage and synovial tissue co-cultures from patients with rheumatoid arthritis (RA) or osteoarthritis (OA).
Figure 7 is a graph of the effect of type I polymerized collagen on the gene expression of cytokines and pro- and anti-inflammatory factors in the co-cultures of cartilage and synovial tissue from patients with rheumatoid arthritis (RA).
Figure 8A is a graph of the effect of type I polymerized collagen on the gene expression of cytokines and pro- and anti-inflammatory factors in the tissues of cartilage and synovial co-cultures from patients with Osteoarthritis (OA).
Figure 8B is a graph of the effect of type I polymerized collagen on cytokine and Foxp3 gene expression in OA.
Figure 9 is a picture of the palliative effect of type I polymerized collagen on histological damage in mice with CIA. Hematoxylin and Eosin and PAS staining. (A) Representative section of the first sacrifice. (B) Representative section of the second sacrifice. The magnification or amplification of the histologies corresponds to 100X.
Figure 10A and 10B is a graph of the effect of type I polymerized collagen in the early (preventive) arthritis model on the percentage of spleen CD4+ T cell subpopulations, in a first and second sacrifice (7 and 56 days) post-Tx, respectively.
Figure 10C and 10D is a graph of the effect of type I polymerized collagen in the long-standing (late or palliative) arthritis model on the percentage of spleen CD4+ T cell subpopulations, in a first and second sacrifice (7 and 35 days) post-Tx, respectively.
Figure 11A is a graph of the effect of type I polymerized collagen on the FACS early CIA model of dendritic cells and splenic lymphocytes in a first sacrifice at 7 days post-Tx.
Figure 11B is a graph of the effect of type I polymerized collagen on the FACS early CIA model of dendritic cells and splenic lymphocytes in a second sacrifice at 56 days post-Tx.
Figure 11C is a graph of the effect of type I polymerized collagen in the FACS advanced CIA model of dendritic cells and splenic lymphocytes in a first sacrifice at 7 days post-Tx.
Figure 11D is a graph of the effect of type I polymerized collagen in the FACS advanced CIA model of dendritic cells and splenic lymphocytes in a second sacrifice at 35 days post-Tx.
Figure 12A is a graph of the effect of type I polymerized collagen in the early (preventive) arthritis model in a first sacrifice at 7 days post-Tx.
Figure 12B is a graph of the effect of type I polymerized collagen in the early (preventive) arthritis model in a second sacrifice at 56 days post-Tx.
Figure 12C is a graph of the effect of type I polymerized collagen in the long-standing model (palliative model) on the percentage of CD11c+ cells of the spleen in a first sacrifice at 7 days post-Tx.
Figure 12D is a graph of the effect of type I polymerized collagen in the long-standing model (palliative model) on the percentage of spleen CD11c+ cells in a second sacrifice at 35 days post-Tx.
Figure 13 is a graphic representation of the symptomatological evaluation of a 60-year-old patient with obesity, passive smoker and diagnosed with COVID-19 (RT-PCR+), with a severity of the disease level 4-5 during treatment using polymerized collagen type I as a function of time.
Figure 14 is a graphic representation of the evaluation of a 55-year-old patient, with hypertension and obesity, with COVID-19 (RT-PCR+), with disease severity level 4-5 during treatment using collagen polymerized type I.
Figure 15 is a graphical representation of the evaluation of a 52-year-old patient, obese and diagnosed with COVID-19 (RT-PCR+), with disease severity level 4-5 during treatment with type I polymerized collagen.
Figure 16 is a graphic representation of the evaluation of a 60-year-old patient, without comorbidities, active smoker and diagnosed with COVID-19 (RT-PCR+), with a severity of the disease level 4-5 during treatment using polymerized collagen type I.
Figure 17 is a graphic representation of the evaluation of a 56-year-old patient, with diabetes and hypertension and diagnosed with COVID-19 (RT-PCR+), with disease severity level 4 during treatment using polymerized collagen type I.
Figure 18 is a graphical representation of the evaluation of the group symptomatological behavior of patients with COVID-19 during treatment (7 days) and post-treatment (7 days) with type I polymerized collagen.
Figure 19 is a graphic representation of the evaluation of the group symptomatological behavior of patients with COVID-19 during treatment (7 days) and post-treatment (7 days) with type I polymerized collagen.
Figure 20 is an overweight 51-year-old woman, without morbidities and diagnosed with COVID-19 (RT-PCR+), with a level 4 disease severity during treatment using type I polymerized collagen.
Figure 21 is a graphical representation of the evaluation of a 51-year-old male patient, without additional morbidities, diagnosed with COVID-19 (RT-PCR+), during treatment using type I polymerized collagen.

### DETAILED DESCRIPTION

The present invention refers to the intramuscular use of type I polymerized collagen to modify post-pneumonia fibrosis due to Covid-19, by preventing acute respiratory failure syndrome, thus preventing the patient from developing fibrotic sequelae, without producing adverse effects.

The use of type I polymerized collagen was evaluated as adjuvant treatment in a pilot trial with two arms, one with intramuscular administration of 1.5 ml of the active ingredient and the other with 1.5 ml vehicle, for 3 days every 12 hours and for 4 days every 24 hours to make a total of 7 days of treatment with a total dose of 15 ml of Collagen-PVP in patients with mild to moderate pneumonia due to Covid-19 (Table 1; categories 1 to 4).

In a second embodiment of the invention, 1.5 ml of the active principle and another with 1.5 ml vehicle are used, for 7 days every 12 hours to make a total of 21 ml of Collagen-PVP in patients with severe to semi-critical pneumonia due to Covid- 19 (Table 1; category 5 to 6).

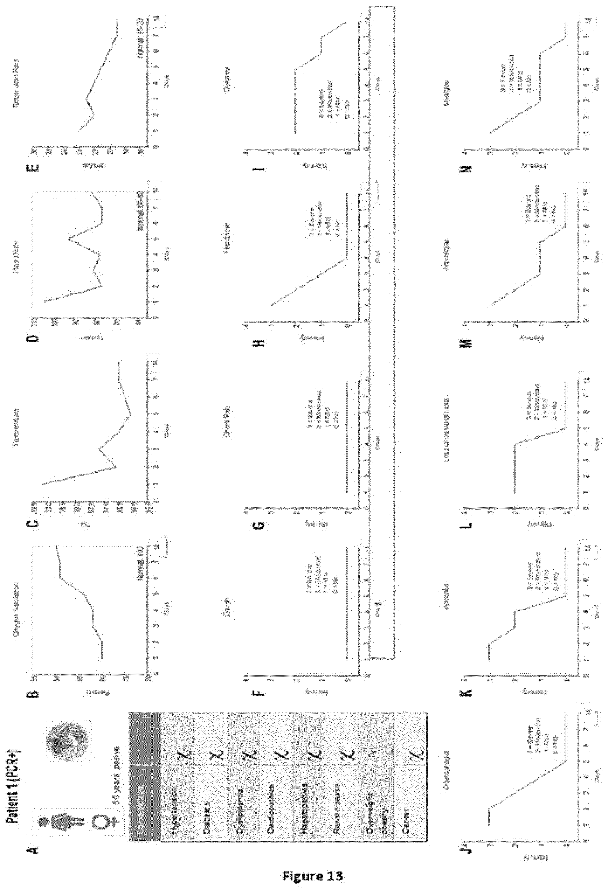

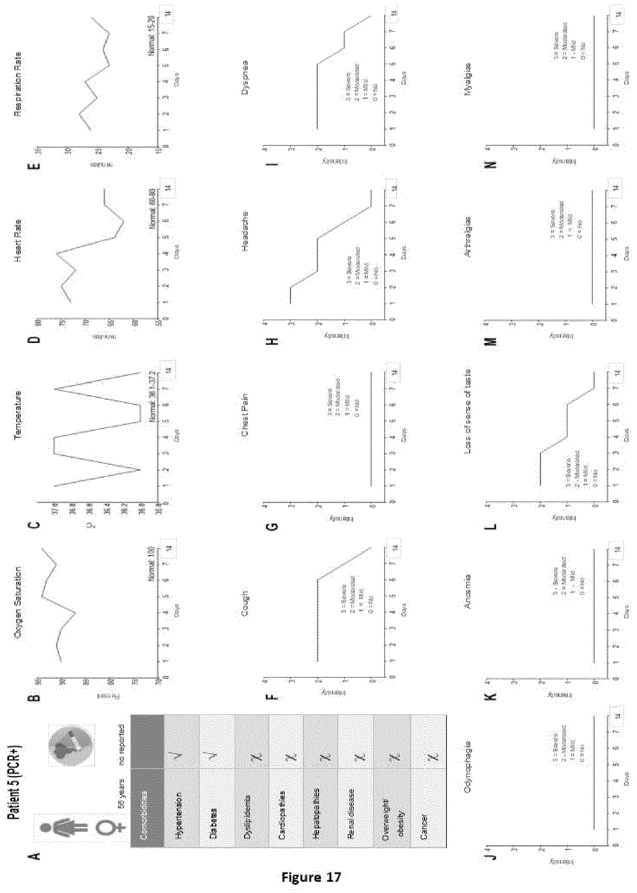

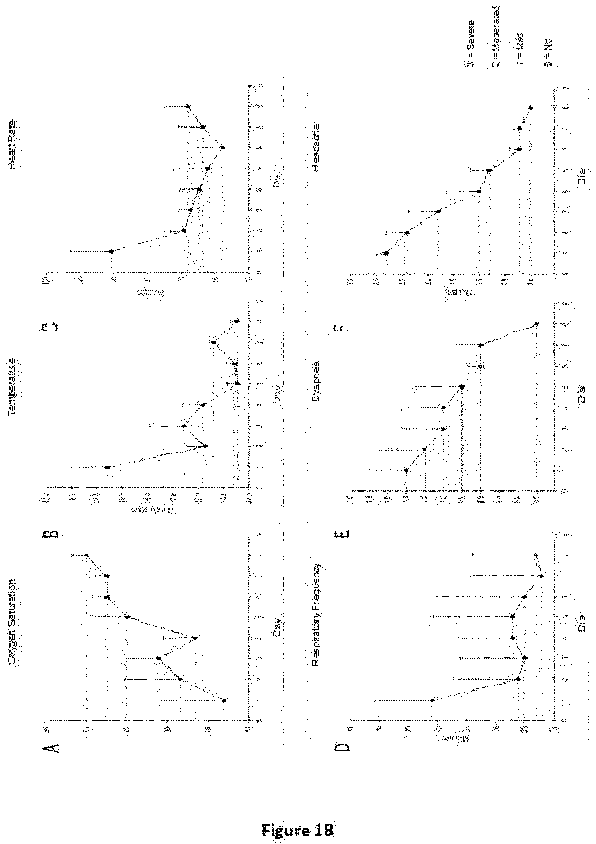

Clinical improvement, changes in the levels of systemic inflammation and soluble pro-inflammatory mediators, as well as fibrosis were evaluated at 7 and 14 days post-treatment. Intramuscular use of polymerized collagen intramuscularly every 12 h for 3 days and then every 24 h for 4 days in patients with mild to severe Covid-19 disease or every 12 h for 7 days in patients with severe to semi-critical disease by Covid-19 is based on the proposed treatment scheme for active rheumatoid arthritis; (Furuzawa-Carballeda J, 2006). In all cases there was a clinical improvement. Clinical improvement was reported individually (Figures 13 to 17) and in groups (Figures 18 to 19), taking into account the following parameters: percentage of SpO2, temperature, heart rate, respiratory rate, cough intervals, chest pain, headache, dyspnea, odynophagia, anosmia, loss of sense of taste, presence of arthralgia and myalgia.

*Description of Symptoms before, during and after treatment* The symptoms were variable in terms of intensity and frequency (Figures 13-17). At the time of diagnosis, all patients had desaturation, headache and respiratory rate changes, 80% had fever, heart rate changes, cough, dyspnea, odynophagia, taste changes, arthralgia and myalgia. Only 40% of the patients manifested anosmia. None of the patients reported chest pain.

Changes in the levels of systemic inflammation and soluble pro-inflammatory mediators, as well as fibrosis were evaluated at 7 and 14 days post-treatment to determine the level of improvement.

### Examples of patients with mild to moderate disease

In 5 patients (3 women), with a mean age of 56.0 ± 3.4 (range: 52-60), with mild-moderate disease, with symptoms, without and with oxygen requirement to maintain SpO₂>92%, with radiographic data or tomography of pulmonary infiltrates, the main comorbidities that represent risk factors for more serious disease were recorded (Figures 13-17) and their clinical evolution was determined through the use of intramuscular collagen-PVP.

### Concomitant medication

Treatment was standardized for all patients with azithromycin 500 mg/24 h; oseltamivir 75 mg/12 h; enoxaparin 40 mg/24 h; adimod 800 mg/12 h; paracetamol 1 gr/8 h; moxifloxacin 400 mg/24 h.

### Administration of type I polymerized collagen

### Description of Symptoms before, during and after treatment

The symptoms were variable in terms of intensity and frequency (Figures 13-17). At the time of diagnosis, all patients had desaturation, headache and respiratory rate changes, 80% had fever, heart rate changes, cough, dyspnea, odynophagia, taste changes, arthralgia and myalgia. Only 40% of the patients manifested anosmia. None of the patients reported chest pain.

With the exception of one patient, who began treatment with type I polymerized collagen on day 5 after the diagnosis of COVID-19, the remainder began on the same day of diagnosis. In all the cases treated with type I polymerized collagen, an increase in SpO₂ (89% to 93%) was determined after 7 days, the disappearance of fever, changes in heart rate and odynophagia. 20% of the patients persisted with alterations in respiratory rate, headache, anosmia, and arthralgia. Cough, myalgia, and loss of taste persisted in 40% of the patients. 60% of the patients persisted with dyspnea (Figures 13-17).

On day 14, that is, 7 days post-treatment with type I polymerized collagen, an increase in pSO₂ was determined (90 to 94%), and in 20% of the patients there were mild alterations in heart rate, cough and loss of the sense of taste. The other symptoms disappeared (Figures 13-17).

**Figure 13****.** Symptomatic evaluation of patients with COVID-19 during treatment using type I polymerized collagen. A) The figure and table belong to a 60-year-old patient diagnosed with COVID-19 (RT-PCR+), passive smoker with obesity and a severity of illness level 4-5. Symptoms such as B) pSO₂, C) temperature, D) heart rate, E) respiratory rate, F) productive or non-productive cough, G) chest pain, H) headache, I) dyspnea, J) odynophagia, K) anosmia, L) loss of the sense of taste, M) arthralgias and N) myalgias, are reported during 14 days after the start of treatment with polymerized collagen type I. The date of diagnosis was 04/29/2020 and the start of treatment with Type I polymerized collagen was on 05/09/2020.

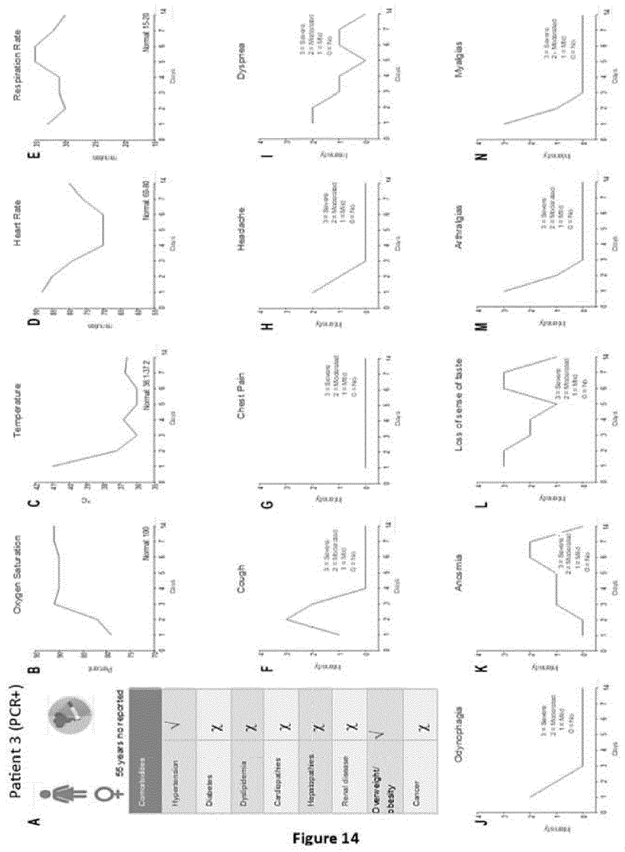

**Figure 14****.** Symptomatic evaluation of patients with COVID-19 during treatment using type I polymerized collagen. **A)** The figure and table belong to a 55-year-old patient diagnosed with COVID-19 (RT-PCR+), with hypertension and obesity, and disease severity level 4-5. Symptoms such as **B)** pSO₂, C) temperature, D) heart rate, E) respiratory rate, F) productive or non-productive cough, G) chest pain, H) headache, I) dyspnea, J) odynophagia, K) anosmia, L) loss of the sense of taste, M) arthralgias and N) myalgias, are reported during 14 days after the start of treatment with polymerized collagen type I. The date of diagnosis was 04/04/2020 and the start of treatment with Type I polymerized collagen was on 05/04/2020.

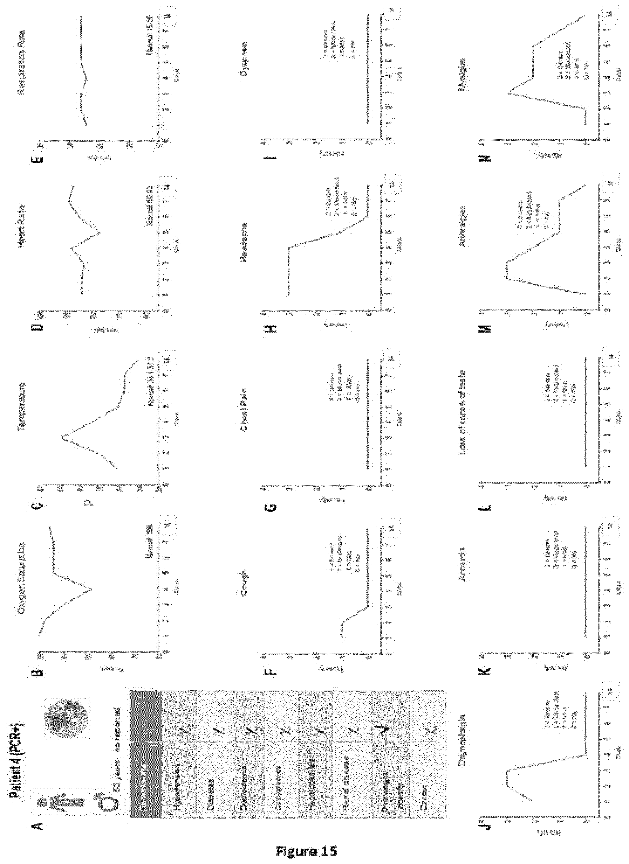

**Figure 15****.** Symptomatic evaluation of patients with COVID-19 during treatment with type I polymerized collagen. **A)** The figure and table belong to a 52-year-old patient diagnosed with COVID-19 (RT-PCR+), with obesity, and a severity of disease level 4-5. Symptoms such as B) pSO₂, C) temperature, D) heart rate, E) respiratory rate, F) productive or non-productive cough, G) chest pain, H) headache, I) dyspnea, J) odynophagia, K) anosmia, L) loss of sense of taste, M) arthralgia and N) myalgia, are reported during 14 days after the start of treatment with polymerized collagen type I. The date of diagnosis was 02/10/2020 and the start of treatment with Type I polymerized collagen was on 05/11/2020.

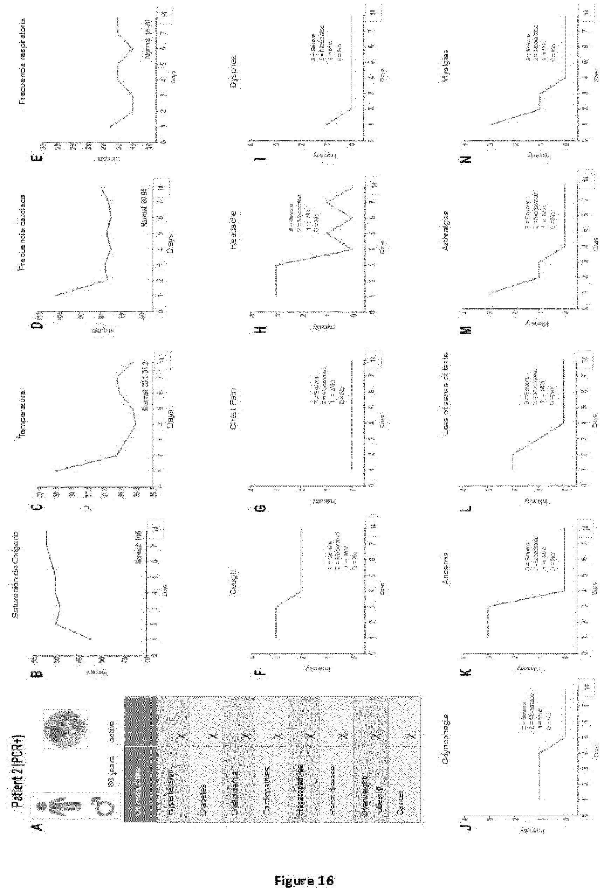

**Figure 16****.** Symptomatic evaluation of patients with COVID-19 during treatment with type I polymerized collagen. **A)** The figure and table belong to a 60-year-old male patient diagnosed with COVID-19 (RT-PCR+), an active smoker without comorbidities, and disease severity level 4-5. Symptoms such as B) pSO₂, C) temperature, D) heart rate, E) respiratory rate, F) productive or non-productive cough, G) chest pain, H) headache, I) dyspnea, J) odynophagia, K) anosmia, L) loss of sense of taste, M) arthralgias and N) myalgias, are reported during 14 days after the start of treatment with polymerized collagen type I. The date of diagnosis was 05/10/2020 and the start of treatment with Type I polymerized collagen was on 05/10/2020.

**Figure 17****.**
Symptomatic evaluation of patients with COVID-19 during treatment using type I polymerized collagen. **A)** The figure and table belong to a 60-year-old patient diagnosed with COVID-19 (RT-PCR+), with diabetes and hypertension, and disease severity level 4. Symptoms such as **B)** pSO₂, C) temperature, D) heart rate, E) respiratory rate, F) productive or non-productive cough, G) chest pain, H) headache, I) dyspnea, J) odynophagia, K) anosmia, L) loss of sense of taste, M) arthralgia and N) myalgia, are reported during 14 days after the start of treatment with polymerized collagen type I. The date of diagnosis was 09/05/2020 and the start of treatment with type I polymerized collagen was on 05/11/2020.

### Adverse effects

No adverse effects were detected, except for pain at the application site, which persisted for 5-15 minutes.

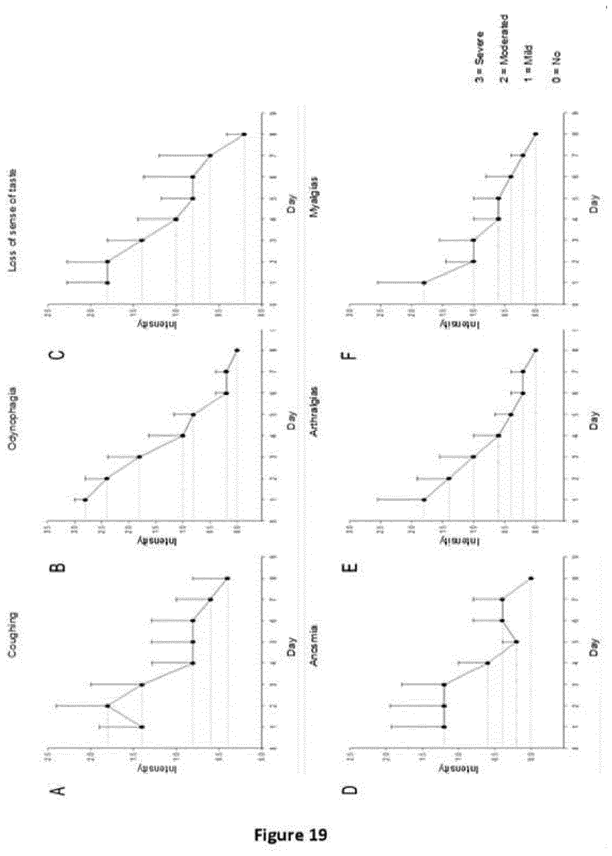

According to all the previous Examples, the use of type I polymerized collagen or collagen-PVP as a therapeutic adjuvant in hyperinflammation and cytokine storm caused by COVID-19 in the group of patients analyzed showed that at the end of treatment and during 7-day follow-up, the patients presented an increase in SpO₂ and none required oxygen to maintain saturation >90% (Figure 18). None of the patients had acute respiratory failure syndrome, nor did they require hospitalization. No patient died. This suggests that type I polymerized collagen exerts a potent systemic inflammatory regulatory effect. No complications related to SARS-CoV2 infection, or due to the use of collagen-PVP, were determined. Dyspnea and headache disappeared in all patients, and respiratory rate and temperature normalized (Figures 18 and 19). Likewise, odynophagia, anosmia, arthralgias and myalgias disappeared (Figure 19), all of the above without producing adverse effects, except for pain at the application site.

**Figure 18****.** Group symptomatic behavior of patients with COVID-19 during treatment (7 days) and post-treatment (7 days) with polymerized collagen type I. **A)** pSO₂, **B)** Temperature, **C)** Heart rate, **D)** Respiratory rate, **E)** Dyspnea, **F)** Headache. Intensity is reported as None = 0, Mild, tolerable = 1, Moderate, bothersome = 2, and Severe, disabling = 3. Data represent the mean ± standard error of treated patients (n = 5).

**Figure 19****.** Group symptomatic behavior of patients with COVID-19 during treatment (7 days) and post-treatment (7 days) with polymerized collagen type I. **A)** Cough, **B)** Odynophagia, **C)** Loss of taste, **D)** Anosmia, **E)** Arthralgia, **F)** Myalgia. Intensity is reported as None = 0, Mild, tolerable = 1, Moderate, bothersome = 2, and Severe, disabling = 3. Data represent the mean ± standard error of treated patients (n = 5) .

### Examples of patients with severe to semi-critical disease

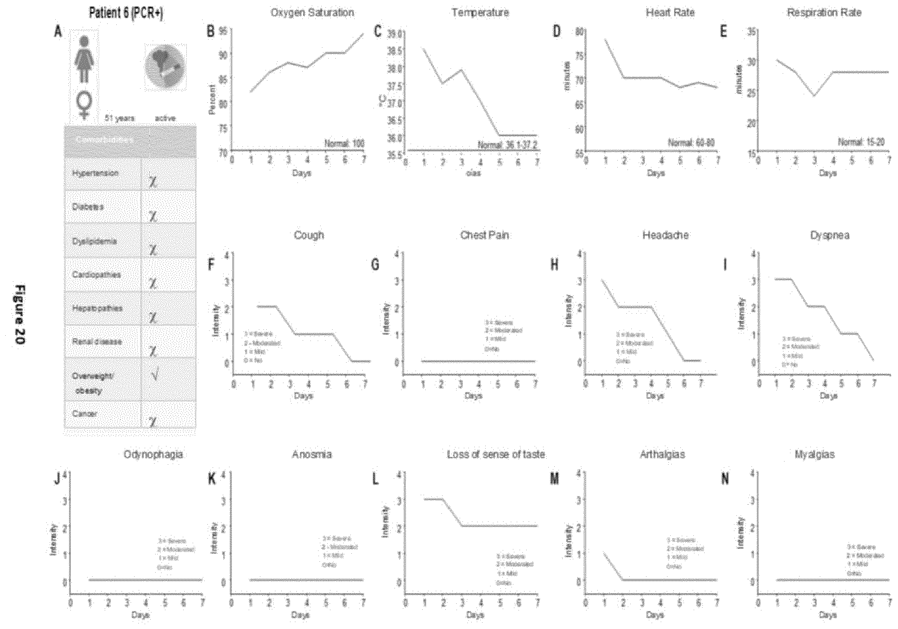

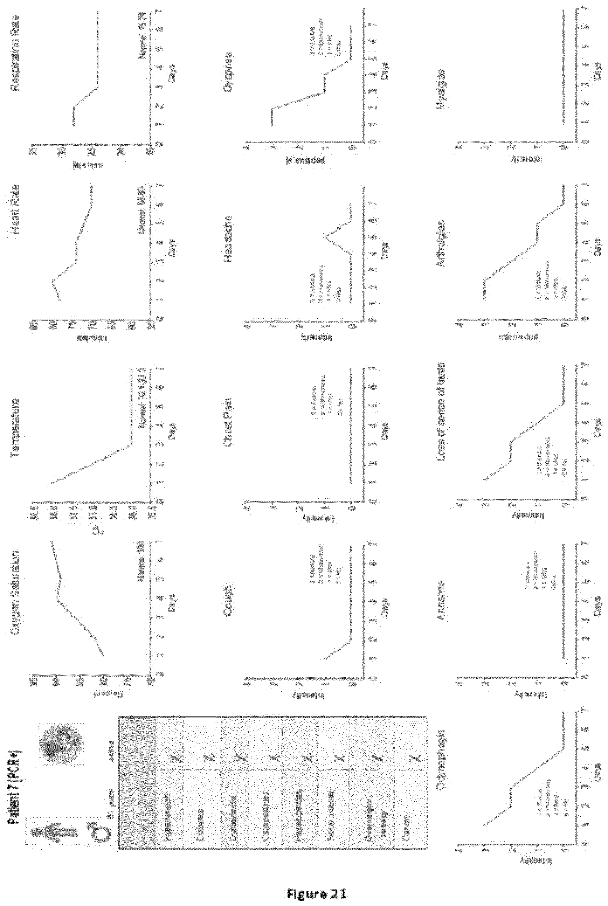

In 2 patients (1 woman), with an average age of 51.0 ± 0.0, with severe-semi-critical disease, with symptoms, with oxygen requirement to maintain SpO₂>92%, with radiographic data of infiltrates in both lungs, the main comorbidities that represent risk factors for more serious disease (Figures 20 and 21) and by using 1.5 ml of intramuscular collagen-PVP every 12 h for 7 days, their clinical evolution was determined.

### Concomitant medication

Treatment was standardized with azithromycin 500 mg/24 h for 6 days; ivermectin 12 mg every 24 hours for 2 days.

### Description of Symptoms before, during and after treatment

The symptoms were variable in terms of intensity and frequency (Figures 20 and 21). At the time of diagnosis, the patients presented desaturation, alterations in respiratory rate, fever, alterations in heart rate, dyspnea, alterations in the sense of taste, and arthralgias.

The patients started treatment with type I polymerized collagen late (between 10 and 15 days after the onset of symptoms).

In both cases treated with type I polymerized collagen, an increase in SpO₂ (between 91 and 94%) was determined after 7 days, the disappearance of fever, alterations in heart rate, respiratory rate, headache and arthralgias.

### Adverse effects

No adverse effects were detected, except for pain at the application site, which persisted for 5-15 minutes.

**Figure 20****.** Symptomatic evaluation of patients with COVID-19 during treatment using type I polymerized collagen. **A)** The figure and table belong to a 51-year-old patient diagnosed with COVID-19 (RT-PCR+), who is overweight, active smoker and disease severity level 6. Symptoms such as **B)** pSO₂, C) temperature, D) heart rate, E) respiratory rate, F) productive or non-productive cough, G) chest pain, H) headache, I) dyspnea, J) odynophagia, K) anosmia, L) loss of sense of taste, M) arthralgia and N) myalgia are reported during the 7 days of treatment with type I polymerized collagen. The date of diagnosis was 18/05/2020 and the start of treatment with type I polymerized collagen was on 06/04/2020.

**Figure 21****.** Symptomatic evaluation of patients with COVID-19 during treatment using type I polymerized collagen. **A)** The figure and table belong to a 51-year-old patient diagnosed with COVID-19 (RT-PCR+), active smoker and disease severity level 6. Symptoms such as **B)** pSO₂, C) temperature, D) heart rate, E) respiratory rate, F) productive or non-productive cough, G) chest pain, H) headache, I) dyspnea, J) odynophagia, K) anosmia, L) loss of sense of taste, M) arthralgia and N) myalgia are reported during the 7 days of treatment with type I polymerized collagen. The date of diagnosis was 05/23/2020 and the start of treatment with type I polymerized collagen was on 06/04/2020.

## Claims

1. Use of type I polymerized collagen every 12 h for 3 days, after 3 days, every 24 h for 4 days to treat cytokine storm, inflammation and pulmonary fibrosis in patients infected by the SARS-CoV2 virus (Covid -19) with mild to moderate disease.

2. Use of type I polymerized collagen every 12 h for 7 days, to treat cytokine storm, inflammation and pulmonary fibrosis in patients infected by the SARS-CoV2 virus (Covid-19) with severe to semi-critical illness.

3. The use of type I polymerized collagen according to claims 1 and 2, wherein the type I polymerized collagen is formulated to be administered intramuscularly.

4. The use of type I polymerized collagen according to claim 1, wherein the type I polymerized collagen is formulated to be administered intramuscularly in a dose of 1.5 ml of the active ingredient for 3 days every 12 hours and for 4 days every 24 hours for a total of 7 days of treatment with a total dose of 15 ml of Collagen-PVP in patients with Covid-19 pneumonia.

5. The use of type I polymerized collagen according to claim 2, wherein the type I polymerized collagen is formulated to be administered intramuscularly in a dose of 1.5 ml of the active ingredient for 7 days every 12 with a total dose of 21 ml of Collagen-PVP in patients with Covid-19 pneumonia.

6. The use of type I polymerized collagen according to claims 1 and 2, to generate changes in the levels of systemic inflammation and soluble pro-inflammatory mediators, as well as fibrosis that can be determined at 7 and 14 days post-treatment.

7. The use of type I polymerized collagen according to claims 1 and 2, wherein each positive COVID-19 patient was evaluated at the time of diagnosis for desaturation, headache and alterations in respiratory rate, fever, alterations in heart rate, cough, dyspnea, odynophagia, alterations in the sense of taste, arthralgia and myalgia, anosmia and chest pain to determine clinical improvement.

8. The use of type I polymerized collagen in accordance with claims 1 and 2, wherein in advanced stages of COVID-19 disease desaturation, headache and alterations in respiratory rate, fever, alterations in heart rate, cough, dyspnea, odynophagia, alterations in the sense of taste, arthralgia and myalgia, anosmia and chest pain were evaluated to determine clinical improvement.

9. The use of type I polymerized collagen according to claims 1 and 2, formulated so as not to produce adverse effects.
